# EUROPÄISCHE PATENTSCHRIFT

(11) **EP 0 999 811 B1**
(45) Veröffentlichungstag und Bekanntmachung des Hinweises auf die Patenterteilung: **15.09.2004**
(21) Anmeldenummer: 99936332.8
(22) Anmeldetag: 27.05.1999
(51) Int. Cl.: A61F 9/007, A61B 1/012

(54) **VORRICHTUNG ZUR AUFWEITUNG UND WIEDERHERSTELLUNG DES TRÄNENKANALS AM MENSCHLICHEN AUGE**
DEVICE FOR EXPANDING AND RECONSTRUCTING THE LACRIMAL DUCT OF A HUMAN EYE
DISPOSITIF POUR ELARGIR ET RECONSTITUER LE CANAL LACRYMAL AU NIVEAU DE L'OEIL HUMAIN

(30) Priorität: 27.05.1998 DE 19823755
(43) Veröffentlichungstag der Anmeldung: 17.05.2000
(73) Patentinhaber: Neuhann, Thomas, 80801 München (DE); HESKE, Norbert, D-82288 Kottgeisering (DE); Hassel, Jörg, 82284 Grafrath (DE)
(72) Erfinder: Neuhann, Thomas, 80801 München (DE); HESKE, Norbert, D-82288 Kottgeisering (DE); Hassel, Jörg, 82284 Grafrath (DE)
(74) Vertreter: Rösler, Uwe, Dipl.-Phys.
(86) Internationale Anmeldenummer: PCT/DE1999/001561
(87) Internationale Veröffentlichungsnummer: WO 1999/060962

(56) Entgegenhaltungen:
- AT-B- 316 011
- DE-A- 19 542 955
- US-A- 5 593 393

## Beschreibung

### Technisches Gebiet

Die Erfindung bezieht sich auf eine Vorrichtung zur Aufweitung und Wiederherstellung des Tränenkanals am menschlichen Auge, durch den Tränenflüssigkeit vom Auge in den Nasenraum gelangt.

### Stand der Technik

Tränenflüssigkeit dient dem menschlichen Auge nicht nur der Befeuchtung der das Auge im vorderen Bereich umgebenden Hornhaut und bewahrt diese vor Austrocknung, sondern erfüllt darüber hinaus Reinigungswirkung, indem beispielsweise Fremdkörper, Bakterien oder anderweitige Verunreinigungen durch die Tränenflüssigkeit vom Auge regelrecht ausgewaschen werden. Überschüssige Tränenflüssigkeit gelangt über den nasalseitig am Auge vorgesehenen Tränenkanal in den Nasenraum, in der die Tränenflüssigkeit durch die Atemluft verdampft und von dieser aufgenommen wird und beispielsweise der Befeuchtung der Atemwege dient.

In Fällen, in denen der Tränenkanal durch innere Verwucherungen oder Ablagerungen teilweise oder vollständig verstopft ist, wird der natürliche Tränenabfluß wenigstens behindert oder kommt vollständig zum Erliegen. In derartigen Fällen sammelt sich Tränenflüssigkeit im Auge, vorzugsweise im Bereich der Unterlider und es bilden sich regelrecht kleine, stationäre Tränenflüssigkeitsseen aus, in denen sich Verunreinigungen ansammeln können, die Infektionsherde für den Augenraum darstellen.

Insbesondere bei älteren Menschen treten derartige Fälle mangelnden Abfluß von Tränenflüssigkeit auf, die entweder medikamentös und/oder operativ behandelt werden können, wobei bei operativen Eingriffen der Tränenkanal wieder freigelegt wird, jedoch bislang nicht gewährleistet werden kann, daß sich der Tränenkanal nach erfolgter Operation nicht wieder zusetzt. Überdies bedeutet ein operativer Eingriff für den Patienten eine hohe Belastung, die mit Schmerzen und insbesondere mit einem langdauernden Heilungs- und Schonungsprozeßes des Auges verbunden ist, so daß der Patient wenigstens vorübergehend über eine eingeschränkte Sehfähigkeit verfügt.

Alkio [1937] hat für das Einlegen eines Metallfadens in den Tränengang behufs Einführung einer Spiralkanüle in den Ductus nasolacrimalis ein biegsames, dünnes Metallrohr entsprechender Stärke in den Tränennasengang eingeführt, an dem sich als Mandrin ein mit einem kleinen Kopf versehener 30 bis 40 cm langer Metallfaden befindet, der mittels speziell zu diesem Zweck verfertigten Hakens aus dem Nasenloch herausgezogen werden muß (USA―Patentschrift Nr.2,154,968).

Der Erfindung liegt die Aufgabe zugrunde, eine Vorrichtung zur Aufweitung und Wiederherstellung des Tränenkanals am menschlichen Auge, durch den Tränenflüssigkeit vom Auge in den Nasenraum gelangt, derart anzugeben, daß die Belastung für einen zu behandelnden Patienten erheblich gesenkt werden kann, so daß ein mit der erfindungsgemäßen Vorrichtung durchzuführender Eingriff ambulant erfolgen kann, ohne daß der Patient, bedingt durch traumatische Irritationen, an dem zu behandelnden Tränenkanal langwierigen Heilungsprozessen ausgesetzt ist. Die Vorrichtung soll überdies dafür Sorge tragen, daß nach Durchführung der Behandlung der Tränenkanal seine vollständige Funktion wiedererhält und zudem eine erneute Zusetzung des Tränenkanals weitgehend ausschließt.

Die Lösung der Erfindung ist im Anspruch 1 angegeben. Den Erfindungsgedanken vorteilhaft weiterbildende Merkmale sind Gegenstand der Unteransprüche.

Erfindungsgemäß sieht die Vorrichtung zur Aufweitung und Wiederherstellung des Tränenkanals am menschlichen Auge, durch den Tränenflüssigkeit vom Auge in den Nasenraum gelangt, eine Kanülenanordnung mit folgenden einzelne Komponenten vor:

Ein erster Hohlkanal ist an seinem distalen Ende mit wenigstens einem Längsschnitt versehen. Ein Mandrin, vorzugsweise selbst als Hohlkanüle ausgebildet, durchsetzt die erste Hohlkanüle und weitet das distale Ende der ersten Hohlkanüle, über die sich der Längsspalt erstreckt, auf. Über die Außenkontur der ersten Hohlkanüle ist ebenso an deren distalem Bereich ein Hohlkörper vorgesehen, der die Außenkontur der ersten Hohlkanüle umschließt und auf dieser verschiebbar angebracht ist.

Schließlich ist eine zweite Hohlkanüle vorgesehen, die über die erste Hohlkanüle schiebbar ist und sich bis zum proximalseitigen Ende des auf der ersten Hohlkanüle aufgebrachten Hohlkörpers erstreckt und diesen gegen proximalseitig axiales Verrutschen sichert.

Die im einzelnen vorstehend genannten Komponenten ergeben eine Kanülenanordnung, mit der es möglich ist, zum einen durch die am nasalseitigen Augenbereich vorgesehene Tränenkanalöffnung in den Tränenkanal einzudringen und den verschlossenen Tränenkanal aufzuweiten. Durch den inneren Kanal des Mandrins ist vorzugsweise eine Beobachtungsoptik geführt sowie ein Spülkanal vorgesehen, so daß visuell an einem entsprechend vorgesehenen Übertragungsmonitor der Einführvorgang der Kanülenanordnung in den Tränenkanal beobachtet und kontrolliert werden kann, wobei die Spülflüssigkeit zum einen den Sichtbereich vor Kanülenspitze freigibt und zum anderen den Durchstoßvorgang innerhalb des Tränenkanals unterstützt.

Die Vorrichtung zeichnet sich jedoch insbesondere dadurch aus, daß am distalen Endbereich der Kanülenanordnung ein Hohlkörper vorgesehen ist, der mit der Kanülenanordnung in den Tränenkanal gezielt verbracht und dort fixiert werden kann. Die Kanülenanordnung ist derart ausgelegt, daß der Hohlkörper, der auf dem Außenumfang der ersten Hohlkanüle im distalen Endbereich aufgeschoben ist, sowohl in Richtung distalem Ende als auch in Richtung proximalem Ende der Kanülenanordnung fixiert ist. Die proximale Fixierung erfolgt mit Hilfe der zweiten Hohlkanüle, die über die erste Hohlkanüle geschoben ist und distalseitig eine Anschlagfläche aufweist, an der der auf die erste Hohlkanüle aufgeschobene Hohlkörper mit seinem proximalseitigen Ende anstößt. Zur Fixierung des Hohlkörpers am distalen Ende der Kanülenanordnung ist die erste Hohlkanüle an der distalen Spitze mit einem Längsspalt versehen und weist überdies vorzugsweise ein in Richtung der distalen Spitze sich verengendes Innenvolumen auf. Wird der Mandrin in die erste Hohlkanüle eingeschoben, so stößt die distale Spitze des Mandrins an die sich verengende konische Innenkontur der ersten Hohlkanüle und drückt die Kanülenhälften an der distalen Spitze auseinander, wodurch sich der Außendurchmesser der ersten Hohlkanüle am distalen Bereich vergrößert. Die Vergrößerung des Außendurchmessers erfolgt derart, daß der auf der ersten Hohlkanüle aufsitzende Hohlkörper nicht über die Spitze der ersten Hohlkanüle geschoben werden kann. Auf diese Weist ist der Hohlkörper sowohl distal- als auch proximalseitig auf der ersten Hohlkanüle gegen Verrutschen gesichert. In diesem Zustand wird die erfindungsgemäße Kanülenanordnung in den zu behandelnden Tränenkanal eingeführt, bis eine gewünschte Position innerhalb des Tränenkanals erreicht ist, so daß beispielsweise der vorzugsweise als Metallspirale ausgebildete Hohlkörper sich längs über den gesamten Tränenkanal erstreckt. In dieser Position wird der innere Mandrin aus der ersten Hohlkanüle entfernt, wodurch die distale Aufweitung auf den normalen Hohlkanülendurchmesser zurückgeht. In einem weiteren Schritt wird die erste Hohlkanüle relativ zur zweiten Hohlkanüle zurückgezogen, wobei der auf der ersten Hohlkanüle aufsitzende Hohlkörper in Form einer Metallspirale am vorgesehenen Ort innerhalb des Tränenkanals verbleibt, zumal der Hohlkörper gegen proximalseitiges Verrutschen, das durch das Herausziehen der ersten Hohlkanüle verursacht werden kann, durch die zweite Hohlkanüle gesichert ist. Erst nach vollständiger Entnahme der ersten Hohlkanüle aus der zweiten Hohlkanüle kann auch die zweite Hohlkanüle aus dem Tränenkanal entnommen werden, so daß lediglich der in den Tränenkanal verbrachte Hohlkörper fest in diesem verbleibt.

Ferner ist es möglich, nach unmittelbarer Entnahme der ersten Hohlkanüle ein Sichtendoskop durch die im Tränenkanal befindliche zweite Hohlkanüle einzubringen, um den richtigen Sitz des Hohlkörpers innerhalb des Tränenkanals zu kontrollieren. Im Falle einer Nachkorrektur ist die erste Hohlkanüle wieder durch die zweite Hohlkanüle in den Tränenkanal und anschließend der Mandrin in die erste Hohlkanüle einzuführen, so daß der Hohlkörper wieder in beide Richtungen entlang der Hohlkanülenanordnung gegen Verrutschen gesichert ist. In diesem Zustand kann der Hohlkörper relativ zum Tränenkanal erneut justiert werden, so daß nach erfolgter Justierung die vorstehend beschriebene Prozedur der Kanülenanordnungs-Entnahme erfolgen kann.

Als besonders vorteilhaft hat sich die Verwendung einer Metallspirale als Hohlkörper erwiesen, zumal die Metallspirale aufgrund ihrer welligen Oberfläche eine gute Fixierung innerhalb des Gewebes im Tränenkanal ermöglicht. Ferner kann durch Erweitern der Windungen der Metallspirale am jeweiligen Spiralenende sowohl der Zufluß vom Tränensack in den Tränenkanal als auch der Abfluß in den Nasenboden verbessert werden. Schließlich ist die Metallspirale axial flexibel, wodurch Krümmungen im Tränenkanal jederzeit überwunden werden bzw. an diese angepaßt werden können. Selbstverständlich sind auch andere Ausführungen des Hohlkörpers denkbar, beispielsweise elastische Röhrchen, die aus Kunststoff, vorzugsweise aus PMMA oder Silikon-Verbindungen gefertigt sind.

### Kurze Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch. Es zeigen:
Fig. 1 perspektivische Darstellung der gesamten Kanülenanordnung,
Fig. 2 distaler Bereich der ersten Hohlkanüle,
Fig. 3 Mandrin sowie,
Fig. 4 zweite Hohlkanüle.

### Kurze Beschreibung eines Ausführungsbeispiels

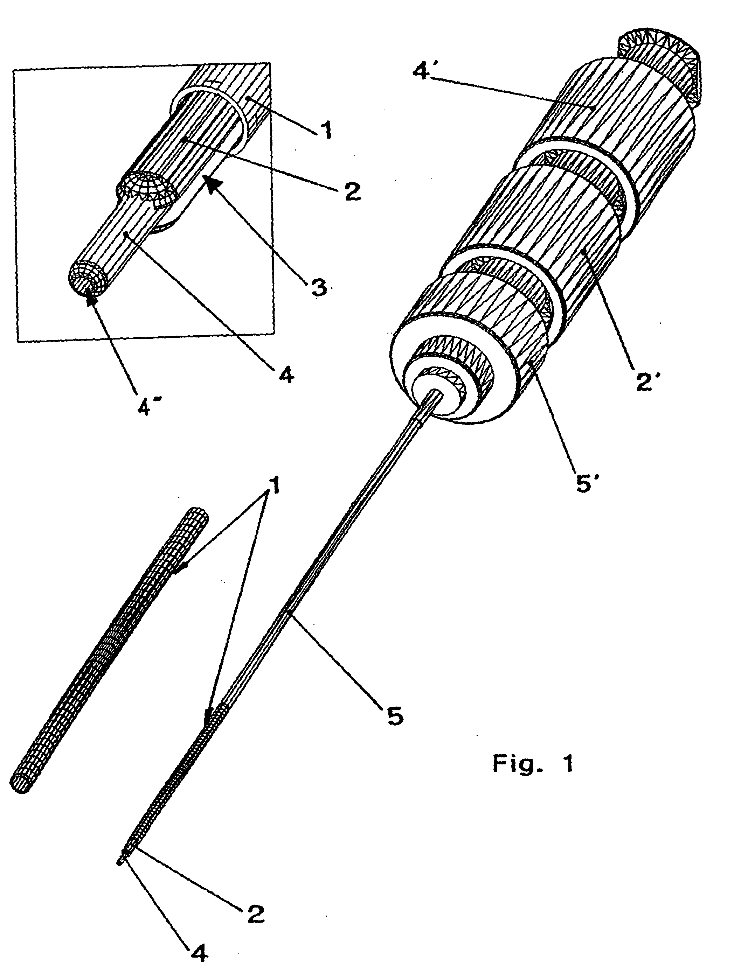

Fig. 1 zeigt eine perspektivische Darstellung der gesamten Kanülenanordnung zur Aufweitung und Wiederherstellung eines Tränenkanals am menschlichen Auge. Ein vorzugsweise als Metallspirale ausgebildeter Hohlkörper 1 sitzt auf der Außenkontur einer ersten Hohlkanüle 2, die an ihrem distalen Ende einen Längsspalt 3 aufweist (siehe hierzu insbesondere die Detaildarstellung in Fig. 1). Durch das Innere der ersten Hohlkanüle 2 ist ein Mandrin 4 geschoben, der seinerseits einen inneren Hohlkanal 4" aufweist, durch den beispielsweise eine Sichtoptik und/oder ein Spülkanal geführt werden kann. Über die erste Hohlkanüle 2 ist ferner eine zweite Hohlkanüle 5 geschoben, deren Längserstreckung derart bemessen ist, daß das distalseitige Ende der zweiten Hohlkanüle 5 mit dem proximalseitigen Ende des distalseitig auf der ersten Hohlkanüle 2 aufsitzenden Hohlkörpers 1 zusammenstößt. Proximalseitig sind die einzelnen Hohlkanülen bzw. der Mandrin mit entsprechenden Handhabungs- bzw. Griffstücken verbunden. Gemäß der Darstellung in Fig. 1 bezeichnet 5' das Griffstück der zweiten Hohlkanüle, 4' das Griffstück des Mandrins sowie 2' das Griffstück der ersten Hohlkanüle.

Die Innen- bzw. Außendurchmesser der beiden Hohlkanülen 2 und 5, des Mandrins 4 sowie des Hohlkörpers 1 sind dabei jeweils derart aufeinander abgestimmt, so daß die einzelnen Komponenten gegeneinander bzw. aufeinander gleiten können.

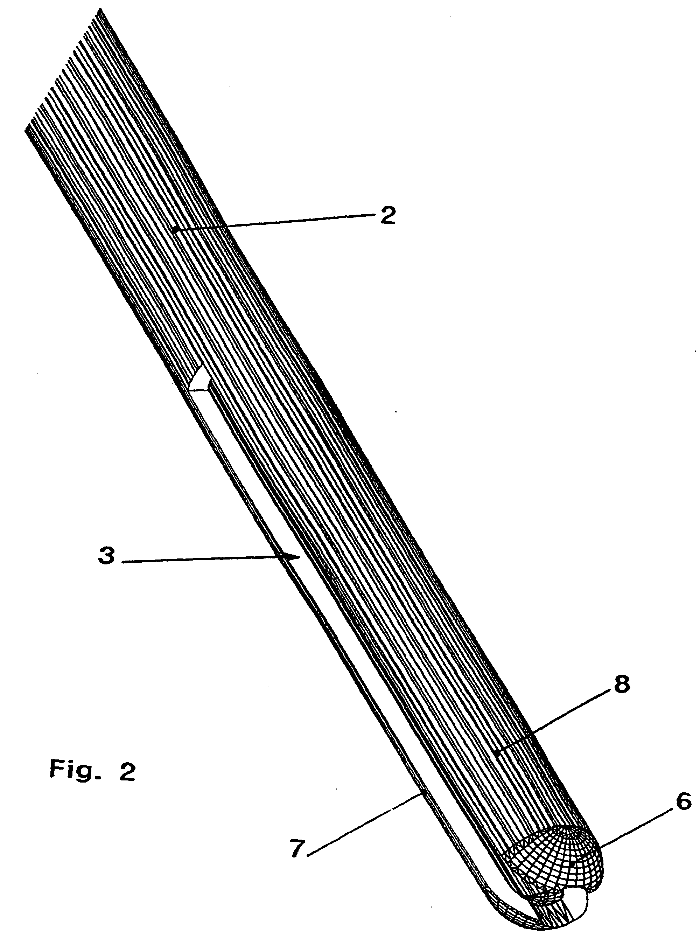

Fig. 2 zeigt den distalen Bereich der ersten Hohlkanüle 2, der über einen Längsspalt 3 verfügt. Insbesondere ist die distale Spitze 6 der ersten Hohlkanüle 2 derart eingebördelt, so daß sich der Innendurchmesser der Hohlkanüle zur distalen Spitze konisch verjüngt. Wird nun der nicht in Fig. 2 dargestellte Mandrin 4 durch die Hohlkanüle 2 geschoben, so stößt der Mandrin 4 auf die sich verengende Innenkontur der distalen Spitze 6 und spreizt dabei die obere und untere Kanülenhälfte 7, 8 voneinander ab. Hierdurch erfährt die erste Hohlkanüle 2 an ihrer distalen Spitze eine Vergrößerung ihres Außendurchmessers, wodurch der auf der Hohlkanüle 2 aufsitzende Hohlkörper nicht über die distale Spitze 6 verschoben werden kann.

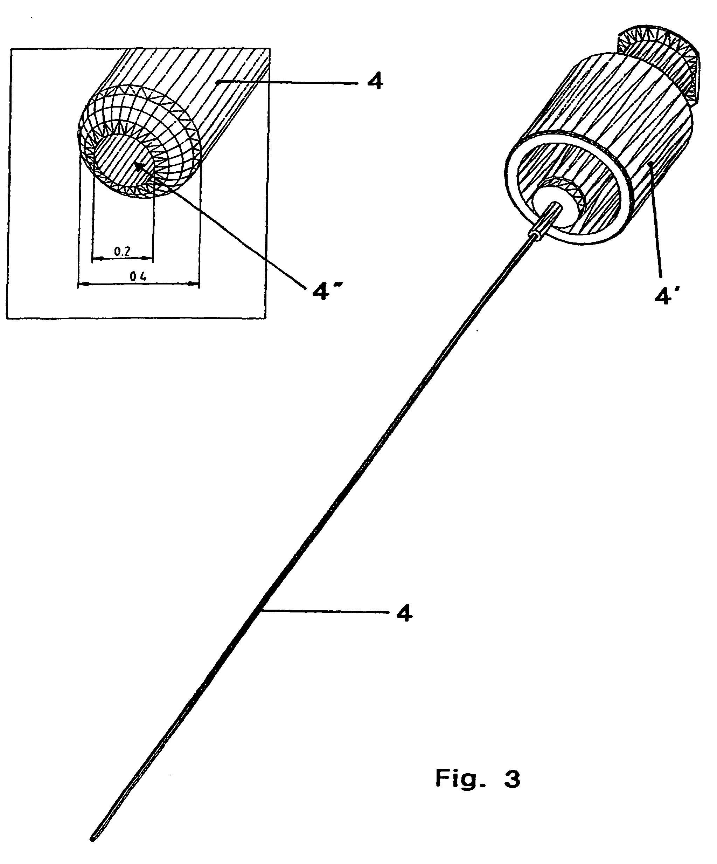

In Fig. 3 ist der Mandrin 4 mit entsprechendem Griffteil 4' dargestellt. In der Detaildarstellung gemäß Fig. 3 geht ein Hohlkanal 4"hervor, durch den, wie vorstehend bereits dargelegt, eine Sichtendoskopoptik in Form einer Glasfaser und/oder ein mit Spülflüssigkeit versehener Kanal durchgeschoben werden kann.

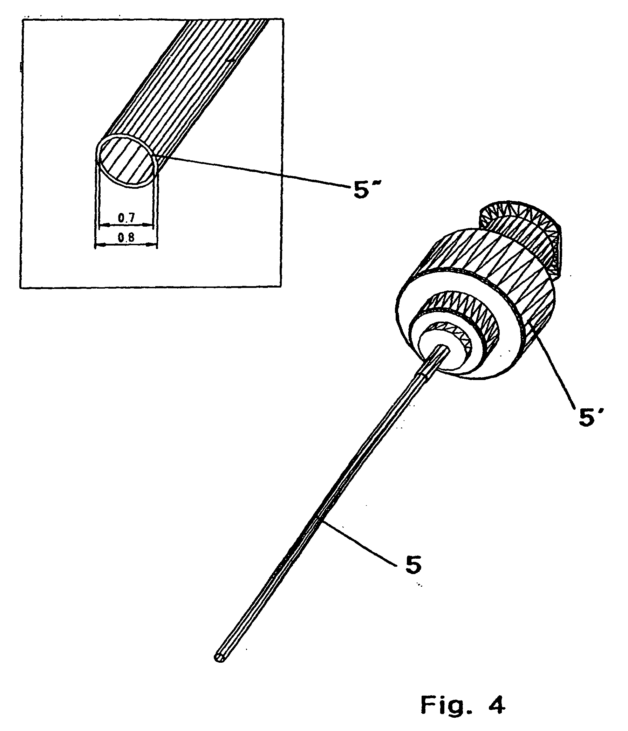

Schließlich ist in Fig. 4 die zweite Hohlkanüle 5 mit entsprechendem Griffteil 5' dargestellt, deren Innendurchmesser derart bemessen ist, daß eine Gleitbewegung auf der ersten Hohlkanüle 2 möglich ist. Zudem verfügt die zweite Hohlkanüle 5 gemäß Detailbilddarstellung in Fig. 4 eine distalseitige Anschlagfläche 5", gegen die der Hohlkörper 1 mit seinem proximalen Ende anstößt.

Vorzugsweise wird der Hohlkörper 1 aus einem Spezialedelstahl gefertigt, aus dem im übrigen auch alle anderen Kanülenbestandteile gefertigt sind. Auch ist es denkbar, Nickel-Titan-Legierungen für die einzelnen Komponenten zu verwenden.

### BEZUGSZEICHENLISTE

- 1: Hohlkörper
- 2: erste Hohlkanüle
- 2': Griffteil der ersten Hohlkanüle
- 3: Längsspalt
- 4: Mandrin
- 4': Griffstück des Mandrins
- 4": Hohlkanal
- 5: zweite Hohlkanüle
- 5': Griffstück der zweiten Hohlkanüle
- 5": Anschlagfläche
- 6: distale Spitze
- 7, 8: untere bzw. obere Hälfte der Hohlkanüle am Längsspalt

## Patentansprüche

1. Vorrichtung zur Aufweitung und Wiederherstellung des Tränenkanals am menschlichen Auge, durch den Tränenflüssigkeit vom Auge in den Nasenraum gelangt, mit
• einer ersten Hohlkanüle (2), deren distales Ende wenigstens einen Längsspalt (3) aufweist,
• einem Mandrin (4), der die erste Hohlkanüle (2) durchsetzt und das distale Ende der ersten Hohlkanüle (2) im Spaltbereich aufweitet,
• einem die Außenkontur der ersten Hohlkanüle (2) im distalen Bereich umschließenden Hohlkörper (1) zur Fixierung im Tränenkanal sowie
• einer zweiten Hohlkanüle (5), die über die erste Hohlkanüle (2) schiebbar ist und sich bis zum proximalseitigen Ende des auf der ersten Hohlkanüle (2) aufgebrachten Hohlkörpers (1) erstreckt und diesen gegen proximalseitig axiales Verrutschen sichert.

2. Vorrichtung nach Anspruch 1,
**dadurch gekennzeichnet, daß** das distale Ende der ersten Hohlkanüle (2) einen den Innendurchmesser der ersten Hohlkanüle (2) einengenden, vorzugsweise konisch einengenden Bereich vorsieht.

3. Vorrichtung nach Anspruch 1 oder 2,
**dadurch gekennzeichnet, daß** die distale Spitze (6) der ersten Hohlkanüle derart eingebördelt ist, daß ihr Innendurchmesser kleiner als der Außendurchmesser des Mandrins (4) ist.

4. Vorrichtung nach einem der Ansprüche 1 bis 3,
**dadurch gekennzeichnet, daß** der Mandrin (4) wenigstens einen Hohlkanal (4") zur Durchführung einer Beobachtungsoptik und/oder einen Spülkanal aufweist.

5. Vorrichtung nach einem der Ansprüche 1 bis 4,
**dadurch gekennzeichnet, daß** der Hohlkörper (1) als Metallspirale ausgebildet ist, dessen Innendurchmesser wenigstens dem Außendurchmesser der ersten Hohlkanüle (2) entspricht und auf die Außenkontur der ersten Hohlkanüle (2) aufschiebbar ist.

6. Vorrichtung nach einem der Ansprüche 1 bis 5,
**dadurch gekennzeichnet, daß** der Hohlkörper (1) eine Länge aufweist, die maximal der Länge des zu behandelnden Tränenkanals entspricht.

7. Vorrichtung nach einem der Ansprüche 1 bis 6,
**dadurch gekennzeichnet, daß** die zweite Hohlkanüle (5) eine ringförmige Anschlagfläche (5") an ihrer distalen Spitze aufweist, an der die proximalseitige Kontur des Hohlkörper (1) anliegt.

8. Vorrichtung nach einem der Ansprüche 1 bis 7,
**dadurch gekennzeichnet, daß** die Aufweitung des distalen Endes (6) der ersten Hohlkanüle (2) derart erfolgt, daß der Außendurchmesser der distalen Spitze (6) der ersten Hohlkanüle (2) größer als der Innendurchmesser des Hohlkörpers (1) ist, wodurch ein distalwärtiges Verrutschen des Hohlkörpers (1) auf der ersten Hohlkanüle (2) vermeidbar ist, und daß bei Entnahme des Mandrins (4) aus der ersten Hohlkanüle (2) die distalseitige Aufweitung auf den ursprünglichen Außendurchmesser der ersten Hohlkanüle (2) zurückgeht, so daß der Hohlkörper (1) distalseitig verbringbar ist.

9. Vorrichtung nach einem der Ansprüche 1 bis 8,
**dadurch gekennzeichnet, daß** proximalseitig zur ersten und zweiten Hohlkanüle sowie zum Mandrin je ein Handhabungsstück (2', 4', 5') angebracht sind.

10. Vorrichtung nach einem der Ansprüche 1 bis 9,
**dadurch gekennzeichnet, daß** der Außendurchmesser der zweiten Hohlkanüle (5) etwa 0,8 mm beträgt.

11. Vorrichtung nach einem der Ansprüche 1 bis 10,
**dadurch gekennzeichnet, daß** der Hohlkörper (1) aus Edelstahl bzw. Implantationsstahl gefertigt ist.

12. Vorrichtung nach einem der Ansprüche 5 bis 11,
**dadurch gekennzeichnet, daß** die Metallspirale an ihren Spiralenden derart aufgeweitet ist, daß der Abstand der Spiralwindungen vergrößert ist.

13. Vorrichtung nach einem der Ansprüche 1 bis 4,
**dadurch gekennzeichnet, daß** der Hohlkörper (1) als Röhrchen ausgebildet ist, dessen Innendurchmesser wenigstens dem Außendurchmesser der ersten Hohlkanüle (2) entspricht und auf die Außenkontur der ersten Hohlkanüle (2) aufschiebbar ist.

14. Vorrichtung nach Anspruch 13,
**dadurch gekennzeichnet, daß** das Röhrchen aus Kunststoff, vorzugsweise aus PMMA oder aus einer Silikon-Verbindung gefertigt ist.

## Claims

1. Device for dilating and restoring the lacrimal duct on the human eye, through which lacrimal fluid flows from the eye into the nasal cavity, comprising
• a first hollow cannula (2) with a distal end which presents at least one longitudinal gap (3),
• a guide (4) passing through said first hollow cannula (2) and having a distal end which dilates the distal end of said first cannula (2) in the region of the gap,
• a hollow body (1) for fixing inside the lacrimal duct, which surrounds the outside contour of said first hollow cannula (2) in the distal zone, as well as
• a second hollow cannula (5) adapted to be pushed over said first hollow cannula (2) and extending up to the end on the proximal side of said hollow body (1), which is placed on said first hollow cannula (2) so as to lock the latter and prevent it from axial sliding on the proximal side.

2. Device according to Claim 1,
**characterised in that** the distal end of said first hollow cannula (2) is provided with a zone which narrows, preferably conically, the inner diameter of said first hollow cannula (2).

3. Device according to Claim 1 or 2,
**characterised in that** the distal tip (6) of said first hollow cannula is so beaded or crimped that its inner diameter is smaller than the outer diameter of said guide (4).

4. Device according to any of the Claims 1 to 3,
**characterised in that** said guide (4) comprises at least one hollow canal (4") for the passage of an optical observation system and/or an irrigation canal.

5. Device according to any of the Claims 1 to 4,
**characterised in that** said hollow body (1) is configured as metal spiral having an inner diameter which corresponds at least to the outer diameter of said first hollow cannula (2) and which is adapted for being pushed onto the outer contour of said first hollow cannula (2).

6. Device according to any of the Claims 1 to 5,
**characterised in that** said hollow body (1) has a length corresponding at maximum to the length of the lacrimal duct to be treated.

7. Device according to any of the Claims 1 to 6,
**characterised in that** said second hollow cannula (5) presents an annular bearing surface (5") on its distal tip, against which the contour of said hollow body (1) on the proximal side bears.

8. Device according to any of the Claims 1 to 7,
**characterised in that** the dilation of the distal end (6) of said first hollow cannula (2) is performed in such a way that the outer diameter of said distal tip (6) of said first hollow cannula (2) is wider than the inner diameter of said hollow body (1) so that sliding of said hollow body (1) on said first hollow cannula (2) towards the distal end can be avoided, and **in that** when said guide (4) is removed from said first hollow cannula (2) the dilation on the distal side is reduced to the original outer diameter of said hollow cannula (2) so that said hollow body (1) can be placed on the distal side.

9. Device according to any of the Claims 1 to 8,
**characterised in that** a respective manipulating element (2', 4', 5') is mounted on the proximal side relative to said first and second hollow cannulae and to said guide.

10. Device according to any of the Claims 1 to 9,
**characterised in that** the outer diameter of said second hollow cannula (5) amounts to 0.8 mm approximately.

11. Device according to any of the Claims 1 to 10,
**characterised in that** said hollow body (1) is made of special steel or implant steel, respectively.

12. Device according to any of the Claims 5 to 11,
**characterised in that** said metal spiral is flared at its spiral ends in such a way that the spacing of the windings of the spiral is increased.

13. Device according to any of the Claims 1 to 4,
**characterised in that** said hollow body (1) is configured as small tube having an inner diameter which corresponds at least to the outer diameter of said first hollow cannula (2) and which is adapted for being pushed onto the outer contour of said first hollow cannula (2).

14. Device according to Claim 13,
**characterised in that** said small tube is made of synthetic material, preferably PMMA or a silicone composition.

## Revendications

1. Dispositif à dilater et rétablir le canal lacrymal à l'oeil humain, par lequel du fluide lacrymal s'écoule à partir de l'oeil dans la cavité nasale, comprenant
• un premier cathéter creux (2) à une extrémité distale, qui présente au moins une fente longitudinale (3),
• un guide (4) passant par ledit premier cathéter creux (2) et ayant une extrémité distale, qui dilate l'extrémité distale dudit premier cathéter (2) dans la zone de la fente,
• un corps creux (1) à se fixer à l'intérieur du canal lacrymal, qui entoure le contour extérieur dudit premier cathéter creux (2) dans la zone distale, ainsi qu'un
• deuxième cathéter creux (5) apte à être poussé sur ledit premier cathéter creux (2) et s'étendant jusqu'à l'extrémité du côté proximal dudit corps creux (1), qui est placé sur ledit premier cathéter creux (2) afin d'arrêter le dernier et d'empêcher son glissement axial du côté proximal.

2. Dispositif selon la revendication 1,
**caractérisé en ce que** l'extrémité distale dudit premier cathéter creux (2) est pourvue d'une zone, qui rétrécit, de préférence en cône, le diamètre intérieur dudit premier cathéter creux (2).

3. Dispositif selon la revendication 1 ou 2,
**caractérisé en ce que** la pointe distale (6) dudit premier cathéter creux est repliée ou sertie d'une telle manière, que son diamètre intérieur soit plus petit que le diamètre extérieur dudit guide (4).

4. Dispositif selon une quelconque des revendications 1 à 3,
**caractérisé en ce que** ledit guide (4) comprend au moins un passage creux (4") pour le passage d'un système d'observation optique et/ou d'un passage d'irrigation.

5. Dispositif selon une quelconque des revendications 1 à 4,
**caractérisé en ce que** ledit corps creux (1) est configuré sous forme d'une spire métallique ayant un diamètre intérieur, qui correspond au moins au diamètre extérieur dudit premier cathéter creux (2) et qui est apte à être poussé sur le contour extérieur dudit premier cathéter creux (2).

6. Dispositif selon une quelconque des revendications 1 à 5,
**caractérisé en ce que** ledit corps creux (1) a une longueur, qui correspond, au maximum, à la longueur du canal lacrymal à traiter.

7. Dispositif selon une quelconque des revendications 1 à 6,
**caractérisé en ce que** ledit deuxième cathéter creux (5) présente une surface d'appui (5") annulaire à sa pointe distale, contre laquelle le contour dudit corps creux (1 ) du côté proximal.

8. Dispositif selon une quelconque des revendications 1 à 7,
**caractérisé en ce que** la dilation de l'extrémité distale (6) dudit premier cathéter creux (2) se fait d'une telle manière, que le diamètre extérieur de ladite pointe distale (6) dudit premier cathéter creux (2) est plus large que le diamètre intérieur dudit corps creux (1) de façon à empêcher le glissement dudit corps creux (1) sur ledit premier cathéter creux (2) vers l'extrémité distale, et **en ce que** quand ledit guide (4) est détaché dudit premier cathéter creux (2), la dilation du côté distal est réduite au diamètre extérieur original dudit cathéter creux (2) d'une telle manière, que ledit corps creux (1) se puisse placer du côté distal.

9. Dispositif selon une quelconque des revendications 1 à 8,
**caractérisé en ce qu'**un élément de maniement respectif (2', 4', 5') est monté du côté proximal relativement auxdits premier et deuxième cathéters creux et relativement audit guide.

10. Dispositif selon une quelconque des revendications 1 à 9,
**caractérisé en ce que** le diamètre extérieur dudit deuxième cathéter creux (5) correspond à 0,8 mm environ.

11. Dispositif selon une quelconque des revendications 1 à 10,
**caractérisé en ce que** ledit corps creux (1) est fait d'acier spécial ou respectivement d'acier d'implant.

12. Dispositif selon une quelconque des revendications 5 à 11,
**caractérisé en ce que** ladite spire métallique converge aux extrémités de ses spires d'une telle manière, que l'écart des tours de la spire s'augmente.

13. Dispositif selon une quelconque des revendications 1 à 4,
**caractérisé en ce que** ledit corps creux (1) est configuré sous forme d'un petit tube ayant un diamètre intérieur, qui correspond au moins au diamètre extérieur dudit premier cathéter creux (2) et qui est apte à être poussé sur le contour extérieur dudit premier cathéter creux (2).

14. Dispositif selon la revendication 13,
**caractérisé en ce que** ledit petit tube est fait en matériau synthétique, de préférence en un composé à PMMA ou à silicone.
